# EUROPEAN PATENT APPLICATION

(11) **EP 4 768 510 A1**
(43) Date of publication of application: **01.07.2026**
(21) Application number: 24932960.8
(22) Date of filing: 31.12.2024
(51) Int. Cl.: C08F 220/34, C08F 220/32, C08F 222/20, C08F 222/14, C08F 222/10, C08K 3/22, C08K 9/06, C08K 5/46, C08K 5/47, A61K 6/887, A61K 6/69, A61K 6/70, A61K 6/62, A61K 6/64, A61K 6/77, A61K 6/818, A61K 6/802

(54) **ANTIBACTERIAL RESIN MIXTURE, AND PREPARATION METHOD THEREFOR AND USE THEREOF**

(30) Priority: 29.03.2024 CN 202410381819; 31.07.2024 CN 202411038365
(71) Applicant: Aidite (Qinhuangdao) Technology Co., Ltd., Qinhuangdao, Hebei 066004 (CN)
(72) Inventor: ZHAO, Haofei, Qinhuangdao, Hebei 066004 (CN); ZHANG, Jiaxin, Qinhuangdao, Hebei 066004 (CN); ZHAO, Lijia, Qinhuangdao, Hebei 066004 (CN); LI, Peng, Qinhuangdao, Hebei 066004 (CN); LI, Hongwen, Qinhuangdao, Hebei 066004 (CN)
(74) Representative: Vogelbruch, Keang
(86) International application number: PCT/CN2024/144665
(87) International publication number: WO 2025/200694

(57) **Abstract**

The present invention relates to the technical field of dental materials, and provides an antibacterial resin mixture, and a preparation method therefor and the use thereof. A resin-infiltrated ceramic composite material prepared by infiltrating the antibacterial resin mixture provided in the present invention into a porous ceramic framework and a dental material prepared from the antibacterial resin mixture with a component (2) can kill oral pathogenic bacteria around a prosthesis and effectively prevent secondary caries. Moreover, compared with existing resin-infiltrated ceramic composite materials, the materials have significantly improved mechanical properties, especially significantly improved fracture load, thereby improving the fracture resistance of the existing resin-infiltrated ceramic composite materials, and have good esthetic properties, thereby prolonging the service life of a prosthesis and enabling a prosthesis material to better utilize the excellent properties thereof during service.

## Description

### CROSS REFERENCE TO THE RELATED APPLICATIONS

The present invention claims priority to Chinese Patent Application No. 2024110383653, entitled "ANTIBACTERIAL RESIN MIXTURE, AND PREPARATION METHOD THEREFOR AND USE THEREOF, AND RESIN-INFILTRATED CERAMIC COMPOSITE MATERIAL AND PREPARATION METHOD HEREFOR", and filed with the China National Intellectual Property Administration on July 31, 2024; and priority to Chinese Patent Application No. 2024103818190, entitled "ANTIBACTERIAL RESIN MIXTURE, AND PREPARATION METHOD THEREFOR AND USE THEREOF, ANTIBACTERIAL RESIN, ELASTIC CERAMIC MATERIAL AND PREPARATION THEREOF, RESIN-INFILTRATED CERAMIC MATERIAL AND PREPARATION THEREOF, AND DENTAL PROSTHESIS", and filed with the China National Intellectual Property Administration on March 29, 2024, the entire contents of which are incorporated herein by reference.

### TECHNICAL FIELD

The present invention relates to the technical field of dental materials, and in particular, to an antibacterial resin mixture, and a preparation method therefor and the use thereof.

### BACKGROUND ART

Dental caries is one of the most common chronic diseases in the human oral cavity, mainly caused by formation of an acidic environment in the oral cavity by human daily dietary habits, which gradually corrodes tooth tissues. Composite materials have mechanical properties closer to those of natural teeth and have become a research hotspot in oral prosthesis. A resin-infiltrated ceramic composite material exhibits more excellent mechanical properties and esthetics.

However, during intraoral wearing, the resin-infiltrated ceramic composite material may also lead to oral diseases due to pathogenic bacterial infections caused by changes in an oral environment, and lead to material breakage due to dietary habits and intraoral chewing conditions.

In view of this, the present invention is proposed.

### SUMMARY

An objective of the present invention is to provide an antibacterial resin mixture, and a preparation method therefor and the use thereof. The resin-infiltrated ceramic material prepared using the antibacterial resin mixture provided in the present invention possesses esthetics and excellent antibacterial properties, and also has significantly improved fracture resistance, which can reduce prosthesis fracture and secondary caries, and prolong the service life of a prosthesis.

To achieve the above objective, the present invention provides the following technical solutions.

An embodiment of the present invention provides an antibacterial resin mixture, wherein the antibacterial resin mixture includes the following component: a component (1) or (2),
wherein the component (1) is composed of, by mass percentage, 1% to 8% of a first antibacterial agent, 0.1% to 3% of an initiator, and the balance being an acrylate monomer; and based on 100% by mass of the first antibacterial agent, the first antibacterial agent is composed of 15% to 25% of an isothiazolinone compound and the balance being a polar organic solvent; and
the component (2) is composed of, by mass percentage, 1% to 10% of a second antibacterial agent and 90% to 99% of a resin matrix; based on 100% by mass of the resin matrix, the resin matrix includes 0.1% to 3% of an initiator and 97% to 99.9% of an acrylate monomer; and based on 100% by mass of the second antibacterial agent, the second antibacterial agent is composed of 15% to 25% of an isothiazolinone compound, 1% to 5% of a coupling-modified nano-metal oxide, and the balance being a polar organic solvent.

An embodiment of the present invention further provides a preparation method for the antibacterial resin mixture as described above, including the following steps: mixing an acrylate monomer, an initiator, and a first antibacterial agent or a second antibacterial agent to obtain the antibacterial resin mixture.

An embodiment of the present invention further provides use of the antibacterial resin mixture as described above or an antibacterial resin mixture prepared by the preparation method described above in preparation of a dental material.

An embodiment of the present invention further provides a resin-infiltrated ceramic composite material, including a porous ceramic framework and an antibacterial resin infiltrated into the porous ceramic framework, wherein the antibacterial resin is formed by curing the antibacterial resin mixture described above or an antibacterial resin mixture prepared by the preparation method described above.

An embodiment of the present invention further provides a preparation method for the resin-infiltrated ceramic composite material as described above, including the following steps: immersing a porous ceramic framework in an antibacterial resin mixture for vacuum infiltration, to infiltrate the antibacterial resin mixture into the porous ceramic framework, and performing curing, to obtain the resin-infiltrated ceramic material; and the antibacterial resin mixture is the antibacterial resin mixture described above or an antibacterial resin mixture prepared by the preparation method described above.

An embodiment of the present invention further provides an antibacterial resin, obtained by curing an antibacterial resin mixture, wherein the antibacterial resin mixture is the antibacterial resin mixture described above or an antibacterial resin mixture prepared by the preparation method described above, wherein the antibacterial resin mixture is prepared from the component (2).

An embodiment of the present invention further provides an elastic ceramic material, including a matrix and a filler dispersed in the matrix, wherein the matrix is the antibacterial resin described above; and the filler includes glass powder.

An embodiment of the present invention further provides a preparation method for the elastic ceramic material described above, including the following steps: mixing an antibacterial resin mixture with a filler to obtain a resin body containing the filler; and
curing the resin body containing the filler to obtain the elastic ceramic material, wherein
the antibacterial resin mixture is the antibacterial resin mixture described above or an antibacterial resin mixture prepared by the preparation method described above.

An embodiment of the present invention further provides a dental prosthesis (dental restoration), made from the resin-infiltrated ceramic composite material described above, the antibacterial resin described above, or the elastic ceramic material described above, wherein the dental prosthesis includes one or more of inlays, onlays, veneers, single crowns, and three-unit bridges, wherein a conventional porous ceramic framework is used in the resin-infiltrated ceramic composite material.

The embodiments of the present invention achieve the following beneficial effects.

The antibacterial resin mixture provided in the embodiments of the present invention uses an isothiazolinone compound as an effective antibacterial ingredient, which can destroy a bacterial structure, hinder bacteria from acquiring nutrients, effectively kill bacteria, reduce plaque accumulation, and enhance an antibacterial capability of the material, and can effectively kill main pathogenic bacteria that cause dental caries and periodontitis, such as *streptococcus mutans* and *porphyromonas gingivalis.* Furthermore, the first antibacterial agent or the second antibacterial agent of the present invention has excellent compatibility with the acrylate monomer, and the antibacterial agent has low viscosity and excellent fluidity, which can enhance a resin infiltration capability and promote a polymerization reaction of a resin system, thereby enhancing mechanical properties of the material, including fracture load, strength, hardness, and elastic modulus. Moreover, a refractive index of the antibacterial agent of the present invention has no significant difference from that of the ceramic framework, so the prepared composite material has no color difference, enhancing esthetic properties of the resin-infiltrated ceramic composite material. According to the present invention, occurrence of secondary caries can be prevented while it is ensured that the resin-infiltrated ceramic composite material effectively exerts excellent performance during service, thereby prolonging the service life of the prosthesis.

### DETAILED DESCRIPTION OF EMBODIMENTS

The technical solutions in implementations of the present invention will be clearly and completely described below in conjunction with the drawings in the implementations of the present invention. Obviously, the described implementations are only part of rather than all of the implementations of the present invention. Based on the implementations in the present invention, all other embodiments obtained by those of ordinary skill in the art without creative efforts shall fall within the protection scope of the present invention.

An embodiment of the present invention provides an antibacterial resin mixture, wherein the antibacterial resin mixture includes the following component: a component (1) or (2),
wherein the component (1) is composed of, by mass percentage, 1% to 8% of a first antibacterial agent, 0.1% to 3% of an initiator, and the balance being an acrylate monomer; and based on 100% by mass of the first antibacterial agent, the first antibacterial agent is composed of 15% to 25% of an isothiazolinone compound and the balance being a polar organic solvent; and
the component (2) is composed of, by mass percentage, 1% to 10% of a second antibacterial agent and 90% to 99% of a resin matrix; based on 100% by mass of the resin matrix, the resin matrix is composed of 0.1% to 3% of an initiator and 97% to 99.9% of an acrylate monomer; and based on 100% by mass of the second antibacterial agent, the second antibacterial agent is composed of 15% to 25% of an isothiazolinone compound, 1% to 5% of a coupling-modified nano-metal oxide, and the balance being a polar organic solvent.

In the present invention, unless otherwise specified, all raw materials used are commercially available products well known in the art.

It should be noted that the coupling-modified nano-metal oxide and triclosan are omitted from the component (1) of the present invention. The coupling-modified nano-metal oxide may reduce fracture load, elastic modulus, and hardness of the resin-infiltrated ceramic composite material, and may also reduce linear transmittance of the composite material, affecting esthetic properties. Although triclosan can achieve a bactericidal effect, it may reduce a degree of resin polymerization and reduce various mechanical properties of the material, especially the fracture load. In the present invention, by omitting the coupling-modified nano-metal oxide and triclosan, the prepared resin-infiltrated ceramic composite material ensures antibacterial and esthetic properties, and also has significantly improved mechanical properties, especially fracture load, which can reduce fracture of the prosthesis and prolong the service life of the prosthesis.

Optionally, in the component (1), by mass percentage, the antibacterial resin mixture provided in the present invention includes 1% to 8% of the first antibacterial agent, optionally 2% to 6%, and optionally 3% to 5%, which is specifically 2%, 5%, or 8% in the embodiments of the present invention. The antibacterial resin mixture includes 0.1% to 3% of an initiator, optionally 0.5% to 2%, and optionally 1.0% to 1.5%.

In the present invention, the first antibacterial agent is composed of an isothiazolinone compound and a polar organic solvent; and mass content of the isothiazolinone compound in the antibacterial agent is 15% to 25%, optionally 17% to 23%, with the balance being the polar organic solvent. In the embodiments of the present invention, the mass content of the isothiazolinone compound in the antibacterial agent is specifically 15%, 20%, or 25%.

Optionally, in the component (2), the second antibacterial agent accounts for 1% to 10%, optionally 2% to 8%, and optionally 4% to 6%. Based on 100% by mass of the resin matrix, the resin matrix includes 97% to 99.9% of the acrylate monomer, optionally 97.5% to 99.5%, and optionally 98% to 99%. Based on 100% by mass of the resin matrix, the resin matrix includes 0.1% to 3% of the initiator, optionally 0.5% to 2.5%, and optionally 1% to 2%. A composite antibacterial agent includes 1% to 5% of the coupling-modified nano-metal oxide, optionally 2% to 4%, and optionally 2.5% to 3.5%.

Optionally, the isothiazolinone compound is one or more of 1,2-benzisothiazolin-3-one (BIT), 2-octyl-4-isothiazolin-3-one (OIT), 2-methyl-4-isothiazolin-3-one (MIT), and 5-chloro-2-methyl-4-isothiazolin-3-one (CMI).

In the present invention, the isothiazolinone compound is innovatively used as an ingredient of the antibacterial agent. The isothiazolinone compound has good compatibility with the resin matrix, which can enhance mechanical properties of the resin material, including fracture load, strength, hardness, and elastic modulus, especially significantly improve the fracture load, and can also promote a polymerization reaction of the acrylate monomer and have a good antibacterial effect.

Optionally, the acrylate monomer includes a main monomer and a diluent monomer.

In the component (1), a mass ratio of the main monomer to the diluent monomer is (1.5 to 10):1, optionally 1.5 to 2.5:1. In the embodiments of the present invention, the mass ratio of the main monomer to the diluent monomer is 1.5:1 or 2.3:1.

In the component (2), a mass ratio of the main monomer to the diluent monomer is (1.5 to 4):1, optionally (2 to 3):1.

Optionally, the main monomer optionally includes one or more of bisphenol A-glycidyl methacrylate (Bis-GMA), ethoxylated bisphenol A dimethacrylate (Bis-MEPP), N,N-dimethylaniline (DMA), and urethane dimethacrylate (UDMA); and the diluent monomer optionally includes one or more of triethylene glycol dimethacrylate (TEGDMA), ethylene glycol dimethacrylate (EGDMA), and trimethylolpropane triacrylate (TMPTMA).

Optionally, the initiator includes a thermal initiator and/or a photoinitiator.

Optionally, the initiator optionally includes a thermal initiator and/or a photoinitiator. The thermal initiator optionally includes one or more of benzoyl peroxide (BPO), tert-butyl peroxyacetate, dicumyl peroxide (DCP), tert-butyl peroxy-2-ethylhexanoate (TBPO), and tert-butyl peroxybenzoate (CP-01). The photoinitiator optionally includes one or more of camphorquinone (CQ), bis(2,4,6-trimethylbenzoyl)phenylphosphine oxide (TPO), and ethyl 2,4,6-trimethylbenzoylphenylphosphinate (TPO-L).

Optionally, the polar organic solvent is an ester solvent.

Optionally, the ester solvent includes an acetate ester. Specifically, the ester solvent is optionally an acetate ester. The acetate ester is optionally one or more of methyl acetate, ethyl acetate, butyl acetate, glycerol monoacetate, glycerol diacetate, and glycerol triacetate.

Optionally, based on 100% by mass of the second antibacterial agent, the second antibacterial agent further includes 0.1% to 0.5% of triclosan, optionally 0.2% to 0.4%, and optionally 0.3%. In the present invention, triclosan, as an organic antibacterial ingredient, has good antibacterial properties.

It should be noted that, in the present invention, there are no special requirements for a preparation method for the coupling-modified nano-metal oxide, as long as a coupling modification method well known in the art is used.

Optionally, the preparation method for the coupling-modified nano-metal oxide includes the following steps: mixing a nano-metal oxide with a coupling agent and a solvent, and performing coupling modification, to obtain the coupling-modified nano-metal oxide.

Optionally, the coupling agent includes a silane coupling agent. The silane coupling agent optionally includes γ-methacryloxypropyltrimethoxysilane (KH570). In the present invention, the solvent optionally includes an ethanol-water mixed solvent; and a volume ratio of ethanol to water in the ethanol-water mixed solvent is optionally (30 to 50): (1 to 5). In the present invention, mass of the coupling agent is optionally 2% to 5% of mass of the nano-metal oxide. In the present invention, there are no special requirements for an amount of solvent used, as long as the nano-metal oxide can be uniformly dispersed.

In the present invention, the coupling modification is optionally carried out under a stirring condition at room temperature, and the coupling modification is optionally carried out for 2 h to 4 h. Upon completion of the coupling modification, in the present invention, the obtained product system is optionally filtered, washed with anhydrous ethanol, and dried to obtain the coupling-modified nano-metal oxide. The nano-metal oxide of the present invention, after coupling modification, can be uniformly dispersed in an oily system, preventing agglomeration, which helps to improve mechanical and esthetic properties of the antibacterial resin.

Optionally, the nano-metal oxide includes one or more of nano-zinc oxide, nano-silver oxide, nano-yttrium oxide, nano-lanthanum oxide, nano-magnesium oxide, nano-copper oxide, nano-alumina, and nano-iron oxide. In the present invention, the nano-metal oxide is used as an inorganic antibacterial ingredient, which is more stable than a metal element, as the metal element is more active and prone to agglomeration.

Optionally, a particle size of the nano-metal oxide ranges from 1 nm to 100 nm, optionally 1 nm to 100 nm, and optionally 20 nm to 80 nm.

It should be noted that in the component (2), the second antibacterial agent uses a metal oxide and an organic compound as release-type and contact-type antibacterial ingredients, respectively. The organic antibacterial ingredient can kill bacteria on a surface of the material, reduce plaque accumulation, and enhance a long-term antibacterial capability of the material. Additionally, release of metal ions kills free bacteria outside the material, forming a multi-antibacterial system, which can effectively kill main pathogenic bacteria that cause dental caries and periodontitis, such as *streptococcus mutans* and *porphyromonas gingivalis.* In addition, in the present invention, the isothiazolinone compound is innovatively used as an ingredient of the antibacterial agent. The isothiazolinone compound has good compatibility with the resin matrix, which can enhance mechanical properties of the resin material and has a good antibacterial effect. Moreover, the composite antibacterial agent of the present invention has lower viscosity than the resin matrix and excellent fluidity, which, upon distribution in the resin matrix, can improve transmittance of the antibacterial resin and enhance esthetic properties of the antibacterial resin.

An embodiment of the present invention further provides a preparation method for the antibacterial resin mixture as described above, including the following steps: mixing an acrylate monomer, an initiator, and a first antibacterial agent or a second antibacterial agent to obtain the antibacterial resin mixture.

Specifically, when the antibacterial resin mixture uses the component (1), a preparation method thereof is as follows: first subjecting the acrylate monomer and the initiator to first stirring under water bath heating at 40°C to 60°C, and then adding the first antibacterial agent for second stirring.

It should be noted that, in the present invention, there are no special requirements for a preparation process of the first antibacterial agent, as long as the isothiazolinone compound is directly fully mixed with the polar organic solvent. Since the isothiazolinone compound itself has certain viscosity, direct addition to the resin monomer may increase viscosity of the resin mixture, which is not conducive to an infiltration process and may degrade performance of formed samples. In the present invention, after the isothiazolinone compound is first mixed with the polar organic solvent, the formed antibacterial agent has lower viscosity, which is conducive to infiltration of the resin mixture, thereby improving performance. Optionally, after mixing, the obtained mixture is subjected to vacuum defoaming. In the present invention, there are no special requirements for an implementation process of vacuum defoaming, as long as a vacuum defoaming process well known in the art is used.

In the present invention, there are no special requirements for the foregoing first stirring and second stirring, as long as uniform mixing of the materials can be ensured. In the embodiments of the present invention, the first stirring is carried out at a speed of 300 r/min for 60 min; and the second stirring is carried out at a speed of 300 r/min for 30 min.

When the antibacterial resin mixture uses the component (2), a preparation method thereof is as follows: first subjecting the acrylate monomer to first stirring under water bath heating at 40°C to 60°C, and then adding the initiator and the second antibacterial agent for second stirring.

It should be noted that, in the present invention, there are no special requirements for a preparation process of the second antibacterial agent, as long as ingredients of the second antibacterial agent are directly fully mixed. In the present invention, the second antibacterial agent is first prepared and then mixed with the acrylic monomer and the initiator, which can ensure uniform dispersion of the nano-metal oxide. If the nano-metal oxide is added directly to the resin matrix, the nano-metal oxide may agglomerate.

In addition, in the present invention, parameters of the first stirring and the second stirring when the antibacterial resin mixture with the component (2) is prepared may be the same as those for preparing the antibacterial resin mixture with the component (1), or may be reasonably adjusted according to an actual situation.

An embodiment of the present invention further provides use of the antibacterial resin mixture as described above or an antibacterial resin mixture prepared by the preparation method described above in preparation of a dental material.

Optionally, the dental material includes an antibacterial resin, an elastic ceramic material, or a resin-infiltrated ceramic composite material.

It should be noted that the dental material prepared by using the antibacterial resin mixture of the present invention has an excellent antibacterial capability and takes into account both mechanical and esthetic properties. The dental material prepared by using the component (1) as a raw material has better fracture resistance. Optionally, the antibacterial resin and the elastic ceramic material in the present invention are both prepared based on the component (2), while the resin-infiltrated ceramic composite material may be prepared by either the component (1) or the component (2). In other implementations of the present invention, the antibacterial resin and the elastic ceramic material may alternatively be prepared based on the component (1).

An embodiment of the present invention further provides a resin-infiltrated ceramic composite material, including a porous ceramic framework and an antibacterial resin infiltrated into the porous ceramic framework. The antibacterial resin is formed by curing the antibacterial resin mixture described above or an antibacterial resin mixture prepared by the preparation method described above.

Optionally, a material of the porous ceramic framework includes any one or a combination of at least two of aluminosilicate, feldspar, silica, zirconia, tungsten oxide, nepheline, and magnesium silicate.

Optionally, the porous ceramic framework includes a coupling-modified porous ceramic framework or a conventional porous ceramic framework.

In the present invention, when the porous ceramic framework is the coupling-modified porous ceramic framework, optionally a porous ceramic framework modified with a silane coupling agent. The silane coupling agent optionally includes γ-methacryloxypropyltrimethoxysilane (KH570). A purpose of using the coupling-modified porous ceramic framework in the present invention is to increase lipophilicity of the ceramic framework, promoting better bonding of the antibacterial resin with the porous ceramic framework.

An embodiment of the present invention further provides a preparation method for the resin-infiltrated ceramic composite material as described above, including the following steps: immersing a porous ceramic framework in an antibacterial resin mixture for vacuum infiltration, to infiltrate the antibacterial resin mixture into the porous ceramic framework, and performing curing, to obtain the resin-infiltrated ceramic material; and
the antibacterial resin mixture is the antibacterial resin mixture described above or an antibacterial resin mixture prepared by the preparation method described above.

It should be noted that, in the present invention, optionally, a porous ceramic framework, i.e., a conventional porous ceramic framework, is first provided. In the present invention, there are no special requirements for the source of the porous ceramic framework, as long as it is prepared by using a method well known in the art or a commercially available product.

When a coupling-modified porous ceramic framework is used to prepare the resin-infiltrated ceramic composite material, in the present invention, optionally, coupling modification is first performed on the porous ceramic framework before immersion. The coupling modification optionally includes the following steps: mixing the porous ceramic framework with a modifying solution, followed by vacuumizing, pressure holding, and heat treatment in sequence, wherein the modifying solution includes an alcohol-water mixed solvent, an acid, and a coupling agent.

In the present invention, alcohol in the alcohol-water mixed solvent optionally includes ethanol, optionally anhydrous ethanol; and a mass ratio of alcohol to water in the alcohol-water mixed solvent is optionally 1: 1. The acid optionally includes any one or a combination of at least two of acetic acid, oxalic acid, hydrochloric acid, citric acid, and phosphoric acid. Mass of the acid is optionally 0.5%0 to 3‰ of mass of the alcohol-water mixed solvent, optionally 1‰ to 2.5‰. The coupling agent optionally includes a silane coupling agent. The type of the coupling agent has been discussed above and is not described in detail herein. Mass of the coupling agent is optionally 1% to 10% of the mass of the alcohol-water mixed solvent, optionally 2% to 6%.

In the present invention, a mass ratio of the porous ceramic framework to the modifying solution is optionally 1:3.

In the present invention, a duration for vacuumizing is optionally 10 min to 30 min; and a duration for pressure holding is optionally 20 min to 60 min. In the present invention, entry of the modifying solution into pores of the porous ceramic framework is facilitated through vacuumizing and pressure holding.

In the present invention, a temperature of the heat treatment is optionally 70°C to 90°C, optionally 80°C, and a temperature holding duration is optionally 3 h to 4 h. In the present invention, grafting of the coupling agent onto the porous ceramic framework is facilitated by heat treatment.

Upon completion of heat treatment, in the present invention, optionally, excess modifying solution is poured off, the modified porous ceramic framework is dried, and then subsequent steps are performed. In the present invention, drying is optionally performed first at 50°C for 1 h to 3 h, and then at 80°C for 2 h to 4 h. In the present invention, the porous ceramic framework that has been dried and modified is utilized.

In an implementation of the present invention, a preparation method for the conventional porous ceramic framework is as follows: pouring 50g of sodium aluminosilicate powder into a ball mill jar, adding 100g of ethanol and 8g of 3% by mass of polyvinyl alcohol aqueous solution for ball milling, wherein a ball milling speed is 600 r/min, and a ball milling duration is 2 h, and then drying obtained ball milling slurry in an oven at 60°C for 10 h; sieving the powder through a 120-mesh stainless steel screen, placing the sieved powder in a molding press, and holding the pressure at 3 MPa for 4 min to obtain a ceramic green body; subjecting the ceramic green body to secondary pressing in a cold isostatic press at 220 MPa, and holding the pressure for 2 min; and placing the ceramic green body after secondary pressing in a sintering furnace, followed by being heated at a rate of 5°C/min to 800°C, holding the pressure for 120 min, and cooling to room temperature with the furnace, to obtain the conventional porous ceramic framework.

A preparation method for the coupling-modified porous ceramic framework is as follows: placing the conventional porous ceramic framework prepared above and a modifying solution in a beaker at a mass ratio of 1:3, vacuumizing in a vacuum box for 20 min, holding the pressure for 20 min, placing in an 80°C oven for 3 h, pouring off the modifying solution, wiping off excess modifying solution from a surface of the ceramic framework, and placing the porous ceramic framework in an oven, first drying at 50°C for 1 h, and then drying at 80°C for 2 h, to obtain the coupling-modified porous ceramic framework.

In the present invention, the porous ceramic framework is immersed in the antibacterial resin mixture for vacuum infiltration, completely infiltrating the antibacterial resin mixture into the porous ceramic framework, and performing curing, to obtain the resin-infiltrated ceramic composite material.

A specific vacuum infiltration process may be reasonably selected according to an actual requirement. The present invention provides two process schemes, which are specifically as follows.

Scheme I: This scheme belongs to stepwise infiltration, which is specifically as follows.

1/3 of a height of the porous ceramic framework is immersed in the antibacterial resin mixture for first vacuum infiltration, the antibacterial resin mixture is supplemented to 2/3 of the height of the porous ceramic framework for second vacuum infiltration, and the antibacterial resin mixture is continuously supplemented to completely immerse the porous ceramic framework for third vacuum infiltration. Pressure for the first vacuum infiltration is optionally 100 Pa to 1000 Pa, optionally 100 Pa to 500 Pa, and optionally 100 Pa to 200 Pa. A duration for the first vacuum infiltration is optionally 24 h to 96 h, optionally 48 h to 96 h, and optionally 72 h to 96 h. Pressure for the second vacuum infiltration is optionally 100 Pa to 1000 Pa, optionally 100 Pa to 500 Pa, and optionally 100 Pa to 200 Pa. A duration for the second vacuum infiltration is optionally 24 h to 96 h, optionally 48 h to 96 h, and optionally 72 h to 96 h. Pressure for the third vacuum infiltration is optionally 0.1 Pa to 100 Pa, optionally 0.1 Pa to 1 Pa. A duration for the third vacuum infiltration is optionally 24 h to 96 h, optionally 48 h to 96 h, and optionally 72 h to 96 h.

It should be noted that stepwise infiltration helps to facilitate better infiltration of the antibacterial resin mixture into the porous ceramic framework.

Scheme II: This scheme belongs to one-step infiltration, which is specifically as follows.

Pressure for vacuum infiltration is optionally -0.1 MPa to 0 MPa, optionally -0.1 MPa. A duration for vacuum infiltration is optionally 48 h to 72 h, optionally 48 h.

In the present invention, there are no special requirements for the above curing condition, as long as a curing condition well known in the art is selected according to the type of the initiator in the antibacterial resin mixture. For example, when the initiator is a photoinitiator, the curing condition is optionally UV light irradiation for 1 min to 5 min; and when the initiator is BPO in a thermal initiator, the curing condition optionally includes: holding at 70°C for 6 h, then heating at a rate of 2 °C/min to 5 °C/min to 100°C, and holding for 6 h.

Optionally, the immersing the coupling-modified porous ceramic framework in an antibacterial resin mixture for vacuum infiltration includes: immersing 1/3 of a height of the coupling-modified porous ceramic framework in the antibacterial resin mixture for first vacuum infiltration, supplementing the antibacterial resin mixture to 2/3 of the height of the porous ceramic framework for second vacuum infiltration, and continuously supplementing the antibacterial resin mixture to completely immerse the porous ceramic framework for third vacuum infiltration,
wherein the antibacterial resin mixture is prepared from the component (1).

Optionally, the first vacuum infiltration is carried out at pressure of 100 Pa to 1000 Pa for 24 h to 96 h; the second vacuum infiltration is carried out at pressure of 100 Pa to 1000 Pa for 24 h to 96 h; and the third vacuum infiltration is carried out at pressure of 0.1 Pa to 100 Pa for 24 h to 96 h.

An embodiment of the present invention further provides an antibacterial resin, obtained by curing an antibacterial resin mixture, with the antibacterial resin mixture being the antibacterial resin mixture described above or an antibacterial resin mixture prepared by the preparation method described above, wherein the antibacterial resin mixture is prepared from the component (2).

It should be noted that before curing, in the present invention, optionally, performing vacuum defoaming on the antibacterial resin mixture with the component (2) is further included. In the present invention, there are no special requirements for a condition of vacuum defoaming, as long as a vacuum defoaming condition well known in the art is used.

In the present invention, there are no special requirements for the curing condition, as long as a curing condition well known in the art is selected according to the type of the initiator in the antibacterial resin mixture. In the present invention, when the initiator is a photoinitiator, the curing condition is optionally UV light irradiation for 1 min to 5 min; and when the initiator is BPO in a thermal initiator, the curing condition optionally includes: holding at 70°C for 6 h, then heating at a rate of 2 °C/min to 5 °C/min to 100°C, and holding for 6 h.

An embodiment of the present invention further provides an elastic ceramic material, including a matrix and a filler dispersed in the matrix. The matrix is the antibacterial resin prepared from the above component (2); and the filler includes glass powder.

In the present invention, mass content of the filler in the elastic ceramic material is optionally 60% to 80%, optionally 65% to 75%, and optionally 68% to 72%. A particle size of the filler is optionally 1 µm to 30 µm, optionally 5 µm to 25 µm, and optionally 10 µm to 20 µm.

In the present invention, the filler includes glass powder; the glass powder optionally includes functional glass powder; and the functional glass powder optionally includes one or more of sodium aluminum silicate powder, boron glass powder, pyrophyllite powder, aluminum silicate fiber powder, silica powder, and spodumene powder.

The present invention further provides a preparation method for the elastic ceramic material in the above solution, including the following steps: mixing the antibacterial resin mixture with the above component (2) with a filler to obtain a resin body containing the filler; and curing the resin body containing the filler, to obtain the elastic ceramic material.

In the present invention, there are no special requirements for a process of mixing the antibacterial resin mixture with the component (2) with the filler, as long as the components can be uniformly mixed. In the present invention, the curing condition is the same as the curing condition for the antibacterial resin, and is not described in detail herein.

An embodiment of the present invention further provides a dental prosthesis, made from the resin-infiltrated ceramic composite material described above, the above antibacterial resin prepared from the component (2), or the elastic ceramic material described above. The dental prosthesis includes one or more of inlays, onlays, veneers, single crowns, and three-unit bridges. A conventional porous ceramic framework is used in the resin-infiltrated ceramic composite material.

In the present invention, there are no special requirements for a preparation method for the dental prosthesis, as long as a preparation method well known in the art is used, which is specifically machining by CAD or CAM system, for example.

The dental prosthesis of the present invention contains the antibacterial resin, and thus exhibits good antibacterial properties and takes into account both mechanical and esthetic properties.

The antibacterial resin mixture and the preparation method and use thereof, and the resin-infiltrated ceramic composite material and the preparation method thereof provided in the present invention will be described in detail below in conjunction with examples, but they cannot be construed as limiting the protection scope of the present invention.

### Example 1

This example provided a resin-infiltrated ceramic composite material. The resin-infiltrated ceramic composite material included a coupling-modified porous ceramic framework and an antibacterial resin infiltrated into the coupling-modified porous ceramic framework. A preparation method for the resin-infiltrated ceramic composite material was as follows.

### Preparation of antibacterial resin mixture:

(1) Bis-GMA was weighed according to Table 1 and placed in a beaker. Then, TEGDMA and BPO were added to the beaker. The mixture was stirred with an electric stirrer at 300 r/min for 60 min under water bath heating at 45°C, to uniformly mix the solution.
(2) Then, the first antibacterial agent was added to the beaker and the resultant was stirred at a speed of 300 r/min for 30 min.
(3) The mixed resin was placed in a vacuum environment to remove bubbles therein, to obtain an antibacterial resin mixture.

### Preparation of coupling-modified porous ceramic framework:

(1) 50 g of sodium aluminosilicate powder was poured into a ball mill jar, and 100 g of ethanol and 8 g of 3% by mass of polyvinyl alcohol aqueous solution were added for ball milling, wherein a ball milling speed was 600 r/min, and a ball milling duration was 2 h, and then the obtained ball milling slurry was dried in an oven at 60°C for 10 h.
(2) The powder was sieved through a 120-mesh stainless steel screen, and the sieved powder was placed in a molding press and held at 3 MPa for 4 min to obtain a ceramic green body. The ceramic green body was subjected to secondary pressing in a cold isostatic press at 220 MPa, and the pressure was held for 2 min.
(3) The ceramic green body after secondary pressing was placed in a sintering furnace, heated at a rate of 5 °C/min to 800°C, held for 120 min, and cooled to room temperature with the furnace, to obtain a porous ceramic framework.
(4) The porous ceramic framework and a modifying solution were placed in a beaker at a mass ratio of 1:3, vacuumized in a vacuum box for 20 min, held for 20 min, and placed in an 80°C oven for 3 h. The modifying solution was poured off, and excess modifying solution on a surface of the ceramic framework was wiped off. The porous ceramic framework was placed in an oven, first dried at 50°C for 1 h, and then dried at 80°C for 2 h to dry the framework, to obtain a modified porous ceramic framework. Composition of the modifying solution was as follows: a mass ratio of alcohol to water was 1:1, mass of acetic acid was 1‰ of mass of the alcohol-water mixed solvent, and mass of KH570 was 5% of the mass of the alcohol-water mixed solvent.

### Preparation of resin-infiltrated ceramic composite material:

(1) 1/3 of a height of the modified porous ceramic framework was immersed in the antibacterial resin mixture, and the modified porous ceramic framework was subjected to low vacuum infiltration at 100 Pa for 72 h; the antibacterial resin mixture was supplemented to immerse 2/3 of the height of the modified porous ceramic framework, and the modified porous ceramic framework was subjected to low vacuum infiltration at 100 Pa for 72 h; and the antibacterial resin mixture was supplemented to completely immerse the modified porous ceramic framework, and the modified porous ceramic framework was subjected to high vacuum infiltration at 1 Pa for 72 h, to obtain infiltrated resin-infiltrated ceramic.
(2) The infiltrated resin-infiltrated ceramic was placed in an autoclave, held at 70°C for 6 h, heated at a rate of 2 °C/min to 100°C, and held for 6 h for curing and molding, to obtain a resin-infiltrated ceramic composite material.

### Examples 2 to 8

In the examples, resin-infiltrated ceramic composite materials were prepared according to component ratios shown in Table 1 and Table 2, and the preparation method was the same as that in Example 1.

**Table 1 Formulations of antibacterial resin mixtures in Examples 1 to 8**

| Component mass percentage group (%) | Example 1 | Example 2 | Example 3 | Example 4 | Example 5 | Example 6 | Example 7 | Example 8 |
|---|---|---|---|---|---|---|---|---|
| Bis-GMA | 58.5 | 56.7 | 54.9 | 28.35 | - | 56.7 | 56.7 | 56.7 |
| Bis-MEPP | - | - | - | - | 56.7 | - | - | - |
| UDMA | - | - | - | 37.8 | - | - | - | - |
| TEGDMA | 39 | 37.8 | 36.6 | 28.35 | 37.8 | - | 37.8 | 37.8 |
| EGDMA | - | - | - | - | - | 37.8 | - | - |
| BPO | 0.5 | 0.5 | 0.5 | 0.5 | 0.5 | 0.5 | 0.5 | 0.5 |
| First antibacterial agent | 2 | 5 | 8 | 5 | 5 | 5 | 5 | 5 |

**Table 2 Composition of first antibacterial agents in Examples 1 to 8**

| Component mass percentage group (%) | Example 1 | Example 2 | Example 3 | Example 4 | Example 5 | Example 6 | Example 7 | Example 8 |
|---|---|---|---|---|---|---|---|---|
| OIT | 15 | 20 | 25 | 20 | 20 | 20 | - | - |
| MIT | - | - | - | - | - | - | 20 | - |
| CMI | - | - | - | - | - | - | - | 20 |
| Glycerol triacetate | 85 | 80 | 75 | 80 | 80 | 80 | 80 | 80 |

### Example 9

This example provided a dental antibacterial resin. A preparation method for the antibacterial resin was as follows.
(1) 29.25 g of Bis-GMA was weighed and placed in a beaker. Then, 29.25 g of TEGDMA, 39 g of UDMA, and 0.5 g of BPO were added to the beaker. The mixture was stirred with an electric stirrer at 300 r/min for 60 min under water bath heating at 45°C, to uniformly mix the monomers.
(2) Then, 2 g of a second antibacterial agent (the composition is shown in Table 3) was added to the beaker and the resultant was stirred at a speed of 300 r/min for 30 min to obtain an antibacterial resin mixture.
(3) The mixed antibacterial resin mixture was placed in a vacuum environment to remove bubbles therein, and then cured and molded according to a heating curve of holding at 70°C for 6 h, heating at a rate of 2°C/min to 100°C, and holding for 6 h, to obtain the dental antibacterial resin.

### Examples 10 to 11

Dental antibacterial resins were prepared according to the component ratios shown in Table 3 and Table 4, and the preparation method was the same as that in Example 9.

### Example 12

This example provided a dental antibacterial elastic ceramic material. The elastic ceramic material used the antibacterial resin in Example 9 as a matrix and glass powder as a filler. A preparation method thereof was as follows.
(1) According to the component ratios of the antibacterial resin mixtures shown in Table 3 and Table 4, an antibacterial resin mixture was obtained by using the same preparation method as in Example 9.
(2) The glass powder and the antibacterial resin mixture were mixed with an electric stirrer stirring at 300 r/min for 60 min. The glass powder accounted for 70% of mass of the elastic ceramic material, and the antibacterial resin matrix accounted for 30% of the mass of the elastic ceramic material.
(3) The mixed antibacterial resin matrix containing glass powder was placed in a mold, and then placed in an autoclave, and heated by holding at 70°C for 6 h, heating at a rate of 2°C/min to 100°C, and holding for 6 h for curing and molding, to obtain the elastic ceramic material.

### Example 13

This example provided a dental antibacterial resin-infiltrated ceramic composite material. The resin-infiltrated ceramic material used a ceramic material as a main body and resin as an auxiliary material. A preparation method thereof was as follows.
(1) According to the component ratios of the antibacterial resin mixtures shown in Table 3 and Table 4, an antibacterial resin mixture was obtained by using the same preparation method as in Example 9.
(2) 50 g of sodium aluminosilicate powder (with a particle size of 1 µm to 10 µm) was poured into a ball mill jar. Then, 100 g of ethanol and 8 g of 3% by mass of polyvinyl alcohol aqueous solution were added for ball milling. A ball milling speed was 600 r/min, and a ball milling duration was 2 h. The obtained ball milling slurry was then dried in an oven at 60°C for 10 h. The powder was sieved through a 120-mesh stainless steel screen. The sieved powder was placed in a molding press and held at 3 MPa for 4 min, to obtain a ceramic green body.
(3) The sodium aluminosilicate ceramic green body was subjected to secondary pressing in a cold isostatic press at 220 MPa, and the pressure was held for 2 min. The ceramic green body after secondary pressing was placed in a sintering furnace, heated at a rate of 5 °C/min to 800°C, held for 120 min, and cooled to room temperature with the furnace, to obtain a conventional porous ceramic green body.
(4) The antibacterial resin mixture was infiltrated in an environment with a vacuum degree of -0.1 MPa for 48 h, and thus the antibacterial resin mixture was completely infiltrated into the ceramic framework, then heated by holding at 70°C for 6 h, heating at a rate of 2 °C/min to 100°C, and holding for 6 h for curing, to obtain the antibacterial resin-infiltrated ceramic material.

### Examples 14 to 17

Dental antibacterial resins were prepared according to the component ratios shown in Table 3 and Table 4, and the preparation method was the same as that in Example 1.

**Table 3 Formulations of antibacterial resin mixtures in Examples 9 to 17**

| Component mass percentage group (%) | Example 9 | Example 10 | Example 11 | Example 12 | Example 13 | Example 14 | Example 15 | Example 16 | Example 17 |
|---|---|---|---|---|---|---|---|---|---|
| Bis-GMA | 29.25 | 28.35 | 26.85 | 28.35 | 28.35 | 56.4 | - | 28.35 | 52.3 |
| Bis-MEPP | - | - | - | - | - | - | 28.35 | - | |
| UDMA | 39 | 37.8 | 35.8 | 37.8 | 37.8 | - | 37.8 | 37.8 | 6 |
| TEGDMA | 29.25 | 28.35 | 26.85 | 28.35 | 28.35 | 37.6 | 28.35 | - | 36.2 |
| EGDMA | - | - | - | - | - | - | - | 28.35 | |
| BPO | 0.5 | 0.5 | 0.5 | 0.5 | 0.5 | 1 | 0.5 | 0.5 | 0.5 |
| Second antibacterial agent | 2 | 5 | 10 | 5 | 5 | 5 | 5 | 5 | 5 |

**Table 4 Composition of second antibacterial agents in Examples 9 to 17**

| Component mass percentage group (%) | Example 9 | Example 10 | Example 11 | Example 12 | Example 13 | Example 14 | Example 15 | Example 16 | Example 17 |
|---|---|---|---|---|---|---|---|---|---|
| Triclosan | 0.3 | 0.5 | 0.1 | 0.5 | 0.5 | 0.2 | 0.5 | 0.4 | - |
| OIT | 15 | 20 | 25 | 20 | 20 | - | - | 15 | 19.6 |
| CMI | - | - | - | - | - | - | 25 | | - |
| MIT | - | - | - | - | - | 18 | - | - | - |
| Modified zinc oxide | 3.7 | 2.5 | 2.2 | 2.5 | 2.5 | - | - | - | 2.4 |
| Modified silver oxide | - | - | - | - | - | 1.8 | 4.5 | 2.6 | - |
| Glycerol triacetate | 76 | 73 | 70.7 | 73 | 73 | 74 | 70 | 77 | 75 |
| Methyl acetate | 5 | 4 | 2 | 4 | 4 | 6 | - | 5 | 3 |

### Comparative Examples 1 to 7

Each comparative example provided an antibacterial resin-infiltrated ceramic composite material. Antibacterial resins in the comparative examples were prepared according to the component ratios shown in Table 5 and Table 6, and the preparation method was the same as that in Example 1.

**Table 5 Formulations of antibacterial resin mixtures in Comparative Examples 1 to 7**

| Component mass percentage group (%) | Comparative Example 1 | Comparative Example 2 | Comparative Example 3 | Comparative Example 4 | Comparative Example 5 | Comparative Example 6 | Comparative Example 7 |
|---|---|---|---|---|---|---|---|
| Bis-GMA | 59.7 | 29.85 | 50.7 | 56.7 | 28.35 | 56.7 | 56.7 |
| UDMA | - | 39.8 | - | - | 37.8 | - | - |
| TEGDMA | 39.8 | 29.85 | 33.8 | 37.8 | 28.35 | 37.8 | 37.8 |
| BPO | 0.5 | 0.5 | 0.5 | 0.5 | 0.5 | 0.5 | 0.5 |
| Antibacterial agent | - | - | 15 | - | - | - | - |
| OIT | - | - | - | 5 | - | - | - |
| Composite antibacterial agent | - | - | - | - | 5 | 5 | 5 |

**Table 6 Composition of composite antibacterial agents in Comparative Examples 5 to 7**

| Component mass percentage group (%) | Comparative Example 5 | Comparative Example 6 | Comparative Example 7 |
|---|---|---|---|
| OIT | 20 | 20 | 20 |
| Modified zinc oxide | 2.5 | 5 | - |
| Triclosan | 0.5 | - | 0.5 |
| MIT | - | - | - |
| CMI | - | - | - |
| Glycerol triacetate | 77 | 75 | 79.5 |

The composition of the antibacterial agent in Comparative Example 3 was the same as that in Example 3. The modified zinc oxide in Table 6 was coupling-modified nano-zinc oxide.

### Comparative Example 8

This comparative example provided a dental antibacterial resin. This comparative example was prepared based on the component ratios shown in Table 7, and the preparation method was the same as that in Example 9.

### Comparative Example 9

This example provided a dental antibacterial elastic ceramic material. The elastic ceramic material used the antibacterial resin in Example 9 as a matrix and glass powder as a filler.

This comparative example was prepared based on the component ratios shown in Table 7, and the preparation method was the same as that in Example 12.

### Comparative Example 10

This comparative example provided a dental antibacterial resin-infiltrated ceramic material. The resin-infiltrated ceramic material used a ceramic material as a main body and resin as an auxiliary material.

This comparative example was prepared based on the component ratios shown in Table 7, and the preparation method was the same as that in Example 13.

### Comparative Examples 11 to 14

These comparative examples provided a dental antibacterial resin. The antibacterial resin included a second antibacterial agent, a resin matrix including a component (2), and an initiator.

Each comparative example was prepared based on the component ratios shown in Table 7, and the preparation method was the same as that in Example 9.

**Table 7 Formulations of antibacterial resin mixtures in Comparative Examples 8 to 14**

| Component mass percentage group (%) | Comparative Example 8 | Comparative Example 9 | Comparative Example 10 | Comparative Example 11 | Comparative Example 12 | Comparative Example 13 | Comparative Example 14 |
|---|---|---|---|---|---|---|---|
| Bis-GMA | 29.85 | 29.85 | 29.85 | 28.35 | 28.35 | 28.35 | 25.35 |
| UDMA | 39.8 | 39.8 | 39.8 | 37.8 | 37.8 | 37.8 | 33.8 |
| TEGDMA | 29.85 | 29.85 | 29.85 | 28.35 | 28.35 | 28.35 | 25.35 |
| BPO | 0.5 | 0.5 | 0.5 | 0.5 | 0.5 | 0.5 | 0.5 |
| Antibacterial agent | - | - | - | - | - | - | 15 |
| Triclosan | - | - | - | 5 | - | - | - |
| OIT | - | - | - | - | 5 | - | - |
| Modified zinc oxide | - | - | - | - | - | 5 | - |

The composition of the antibacterial agent in Comparative Example 14 was the same as that in Example 10.

### Performance Testing

Antibacterial rates of the products prepared in Examples 1 to 17 and Comparative Examples 1 to 14 of the present invention against *streptococcus mutans* and *porphyromonas gingivalis* were tested according to the standard *JC*/*T 897-2014 Antibacterial Property of Antibacterial Ceramics.* Fracture load of the resin-infiltrated ceramic composite materials prepared in Examples 1 to 8 and Comparative Examples 1 to 7 was tested using a Shimadzu universal testing machine. Flexural strength and elastic modulus of the products prepared in Examples 1 to 17 and Comparative Examples 1 to 14 were tested using a Shimadzu universal testing machine according to the standard *GB 30367-2013 Dentistry Ceramic materials.* Hardness of the products prepared in Examples 1 to 17 and Comparative Examples 1 to 14 was tested using a Vickers hardness tester. Light transmittance of the products prepared in Examples 1 to 17 and Comparative Examples 1 to 14 was tested using a haze meter according to the standard *YY 0271.2-2009 Dental water-based cements* to verify esthetic properties of the materials. Test results are summarized in Table 8 to Table 11.

**Table 8 Performance data of resin-infiltrated ceramics in Examples 1 to 8**

| Test item group | | Example 1 | Example 2 | Example 3 | Example 4 | Example 5 | Example 6 | Example 7 | Example 8 |
|---|---|---|---|---|---|---|---|---|---|
| Antibacterial rate (%) | *Streptococcus mutans* | 96.27 | 99.09 | 99.99 | 99.99 | 99.99 | 99.99 | 97.82 | 98.22 |
| | *Porphyromonas gingivalis* | 97.46 | 99.32 | 99.99 | 99.99 | 99.99 | 99.99 | 95.93 | 96.07 |
| Linear transmittance (%) | | 60.8 | 60.5 | 59.4 | 60.0 | 59.8 | 59.6 | 58.6 | 59.2 |
| Flexural strength (MPa) | | 200.2 ±9.6 | 207.3 ±9.4 | 198.8 ±9.2 | 199.8 ±10.1 | 200.7 ±8.3 | 202.2 ±10.3 | 189.4 ±10.1 | 192.2 ±9.6 |
| Elastic modulus (GPa) | | 26.6±0.14 | 28.5±0.15 | 25.6±0.21 | 27.9±0.11 | 28.0±0.22 | 28.1±0.14 | 25.5 ±0.13 | 26.0 ±0.14 |
| Hardness (HV) | | 177.3 | 200.4 | 192.8 | 198.5 | 198.7 | 199.2 | 175.2 | 178.3 |
| Fracture load (N) | | 8277.82 | 8433.21 | 7988.93 | 8374.48 | 8345.23 | 8405.12 | 6810.41 | 7001.32 |

**Table 9 Performance data of resin-infiltrated ceramics in Comparative Examples 1 to 7**

| Test item group | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| Antibacterial rate (%) | *Streptococcus mutans* | 0.22 | 0.11 | 99.99 | 99.99 | 99.99 | 99.99 | 99.99 |
| | *Porphyromonas gingivalis* | 0.17 | 0.23 | 99.99 | 99.99 | 99.99 | 99.99 | 99.99 |
| Linear transmittance (%) | | 55.4 | 54.9 | 57.3 | 57.7 | 54.2 | 47.4 | 59.8 |
| Flexural strength (MPa) | | 185.2 ±8.6 | 182.5 ±11.3 | 175.9 ±12.2 | 169.4 ±11.1 | 179.8 ±10.2 | 186.8 ±10.2 | 162.7 ±8.5 |
| Elastic modulus (GPa) | | 24.4 ±0.11 | 23.8 ±0.12 | 21.8 ±0.21 | 21.1 ±0.22 | 23.1 ±0.11 | 23.9 ±0.11 | 22.8 ±0.22 |
| Hardness (HV) | | 150.5 | 142.2 | 169.4 | 151.7 | 144.5 | 152.5 | 146.7 |
| Fracture load (N) | | 5984.74 | 5297.79 | 6116.04 | 5044.86 | 3346.87 | 3624.77 | 3014.96 |

Analysis of the results in Examples 1 to 4 and Comparative Examples 1 to 2 shows that, compared with no addition of the antibacterial agent, the resin-infiltrated ceramic composite material prepared in the present invention has antibacterial rates over 95% against oral pathogenic bacteria, such as *streptococcus mutans* and *porphyromonas gingivalis.* When the amount of the antibacterial agent added is increased, the antibacterial rate of the antibacterial resin-infiltrated ceramics is over 99%, all exhibiting excellent antibacterial properties. Moreover, the addition of the antibacterial agent significantly enhances mechanical properties of the resin-infiltrated ceramics, including fracture load, elastic modulus, hardness, and strength, especially the fracture load is significantly improved. However, when the proportion of the antibacterial agent added is excessively high, the mechanical properties of the resin-infiltrated ceramics are reduced (see Comparative Example 3).

Analysis of Example 4 and Comparative Example 5 shows that the resin-infiltrated ceramic prepared with OIT compounded with glycerol triacetate alone has higher mechanical properties than that prepared with OIT, modified zinc oxide, and triclosan compounded with glycerol triacetate, including fracture load, elastic modulus, and hardness, especially the fracture load is greatly increased. At the same time, the linear transmittance is improved, enhancing esthetics. This is mainly because zinc oxide hinders light transmission, and a phenolic structure of triclosan reduces a degree of resin polymerization. Under a combined action of the two, a promoting effect of OIT is greatly weakened, ultimately reducing mechanical and esthetic properties of the material.

Analysis of Example 2 and Comparative Example 4 shows that using the antibacterial component OIT alone without addition of the polar organic solvent significantly reduces esthetic properties and mechanical properties (including fracture load, flexural strength, elastic modulus, and hardness) of the resin-infiltrated ceramic composite material. Analysis of Example 2 and Comparative Examples 6 to 7 shows that the addition of the metal oxide zinc oxide significantly reduces hardness, elastic modulus, fracture load, and linear transmittance of the material, affecting the esthetic properties of the material, and the addition of triclosan significantly reduces strength, hardness, elastic modulus, and fracture load of the material. Analysis of Example 2 and Examples 7 to 8 shows that OIT is more compatible with the resin system of the present invention compared to other isothiazolinones such as MIT and CMI, exhibiting better antibacterial properties and mechanical properties.

**Table 10 Performance data of dental materials prepared in Examples 9 to 17**

| Test item group | | Example 9 | Example 10 | Example 11 | Example 12 | Example 13 | Example 14 | Example 15 | Example 16 | Example 17 |
|---|---|---|---|---|---|---|---|---|---|---|
| Antibacterial rate (%) | *Streptococcus mutans* | 95.9 | 99.9 | 99.9 | 99.9 | 99.9 | 99.9 | 99.9 | 99.9 | 99.99 |
| | *Porphyromonas gingivalis* | 96.7 | 99.9 | 99.9 | 99.9 | 99.9 | 99.9 | 99.9 | 99.9 | 99.99 |
| Linear transmittance (%) | | 90.8 | 90.4 | 90.1 | 60.1 | 54.7 | 90.4 | 89.4 | 89.8 | 90.1 |
| Flexural strength (MPa) | | 190.5 ±10.6 | 189.3 ±9.4 | 187.6 ±10.2 | 252.3±9.6 | 182.4±10. 4 | 188.7±8.3 | 190.3±9.6 | 188.2±8.5 | 189.7±9.6 |
| Elastic modulus (GPa) | | 4.3 ±0.18 | 4.2 ±0.14 | 4.0 ±0.2 | 15.2±0.13 | 23.1±0.11 | 4.1±0.16 | 4.3± 0.11 | 4.2± 0.11 | 4.1± 0.18 |
| Hardness (HV) | | 84.8 | 84.3 | 83.6 | 102.1 | 142.3 | 84.1 | 84.9 | 83.9 | 84.2 |

**Table 11 Performance data of dental materials prepared in Comparative Examples 1 to 7**

| Test item group | | Comparative Example 8 | Comparative Example 9 | Comparative Example 10 | Comparative Example 11 | Comparative Example 12 | Comparative Example 13 | Comparative Example 14 |
|---|---|---|---|---|---|---|---|---|
| Antibacterial rate (%) | *Streptococcus mutans* | 0.3 | 0.2 | 0.2 | 83.7 | 90.9 | 87.5 | 99.9 |
| | *Porphyromonas gingivalis* | 0.6 | 0.3 | 0.1 | 79.4 | 89.7 | 85.3 | 99.9 |
| Linear transmittance (%) | | 88.3 | 58.6 | 53.6 | 86.2 | 87.6 | 67.4 | 86.8 |
| Flexural strength (MPa) | | 180.3 ±8.7 | 250.4±9.3 | 181.6±8.2 | 153.5 ±12.3 | 170.9 ±10.2 | 166.4 ±14.1 | 168.2 ±11.4 |
| Elastic modulus (GPa) | | 4.4 ±0.21 | 14.5±0.14 | 22.4±0.13 | 2.9 ±0.22 | 3.3 ±0.11 | 3.9 ±0.26 | 3.8 ±0.17 |
| Hardness (HV) | | 83.2 | 100.7 | 139.2 | 72.2 | 78.4 | 80.1 | 81.5 |

Analysis of the results in Examples 9 to 17 and Comparative Examples 8 to 10 shows that the antibacterial resin, antibacterial elastic ceramic, and antibacterial resin-infiltrated ceramic prepared in the present invention have antibacterial rates over 95% against oral pathogenic bacteria, such as *streptococcus mutans* and *porphyromonas gingivalis.* When the amount of the composite antibacterial agent added is increased, the antibacterial rate of the antibacterial resin is over 99%, all exhibiting excellent antibacterial properties. Moreover, the addition of the composite antibacterial agent enhances mechanical properties and esthetic properties of the three dental materials.

Analysis of the results in Comparative Examples 11 to 13 and Examples 8 to 10 shows that, compared with the multi-antibacterial system formed by multiple antibacterial components of the present invention, the antibacterial rate of a single component is lower, and the addition of the single component significantly reduces the linear transmittance, strength, and elastic modulus of the resin material, i.e., reduces esthetic and mechanical properties of the material.

Analysis of the results in Comparative Example 14 and Example 10 shows that when the amount of the second antibacterial agent added is excessively high, the antibacterial resin will lose some of its own properties, reducing mechanical and esthetic properties of the material.

With reference to the data results in Table 8 to Table 11, it can also be concluded that the coupling-modified nano-metal oxide and triclosan are omitted from the component (1) in the antibacterial resin mixture in the present invention. The coupling-modified nano-metal oxide will reduce the fracture load, elastic modulus, and hardness of the resin-infiltrated ceramic composite material, and also reduce linear transmittance of the composite material, affecting esthetic properties. Although triclosan can achieve a bactericidal effect, it will reduce a degree of resin polymerization and reduce various mechanical properties of the material, especially the fracture load. In the present invention, by omitting the coupling-modified nano-metal oxide and triclosan, the prepared resin-infiltrated ceramic composite material ensures antibacterial and esthetic properties, and also has significantly improved mechanical properties, especially fracture load, which can reduce fracture of the prosthesis and prolong the service life of the prosthesis.

The above are only optional implementations of the present invention. It should be noted that for those of ordinary skill in the art, several improvements and modifications can be made without departing from the principles of the present invention, and these improvements and modifications should also be regarded as falling within the protection scope of the present invention.

### INDUSTRIAL APPLICABILITY

The resin-infiltrated ceramic material prepared using the antibacterial resin mixture provided in the present invention possesses esthetics and excellent antibacterial properties, and also has significantly improved fracture resistance, which can reduce prosthesis fracture and secondary caries, and prolong the service life of a prosthesis.

## Claims

1. An antibacterial resin mixture, **characterized in that** the antibacterial resin mixture comprises following component: a component (1) or (2),
wherein the component (1) is composed of, by mass percentage, 1% to 8% of a first antibacterial agent, 0.1% to 3% of an initiator, and a balance being an acrylate monomer; and based on 100% by mass of the first antibacterial agent, the first antibacterial agent is composed of 15% to 25% of an isothiazolinone compound and a balance being a polar organic solvent; and
the component (2) is composed of, by mass percentage, 1% to 10% of a second antibacterial agent and 90% to 99% of a resin matrix; based on 100% by mass of the resin matrix, the resin matrix is composed of 0.1% to 3% of an initiator and 97% to 99.9% of an acrylate monomer; and based on 100% by mass of the second antibacterial agent, the second antibacterial agent is composed of 15% to 25% of an isothiazolinone compound, 1% to 5% of a coupling-modified nano-metal oxide, and a balance being a polar organic solvent.

2. The antibacterial resin mixture according to claim 1, wherein the isothiazolinone compound is one or more of 1,2-benzisothiazolin-3-one, 2-octyl-4-isothiazolin-3-one, 2-methyl-4-isothiazolin-3-one, and 5-chloro-2-methyl-4-isothiazolin-3-one.

3. The antibacterial resin mixture according to claim 1, wherein the acrylate monomer comprises a main monomer and a diluent monomer;
in the component (1), a mass ratio of the main monomer to the diluent monomer is (1.5 to 10):1; and in the component (2), a mass ratio of the main monomer to the diluent monomer is (1.5 to 4):1;
optionally, the main monomer is one or more of bisphenol A-glycidyl methacrylate, ethoxylated bisphenol A dimethacrylate, N,N-dimethylaniline, and urethane dimethacrylate; and
the diluent monomer is one or more of triethylene glycol dimethacrylate, ethylene glycol dimethacrylate, and trimethylolpropane triacrylate.

4. The antibacterial resin mixture according to claim 1, wherein the initiator comprises a thermal initiator and/or a photoinitiator;
optionally, the thermal initiator comprises one or more of benzoyl peroxide, tert-butyl peroxyacetate, dicumyl peroxide, tert-butyl peroxy-2-ethylhexanoate, and tert-butyl peroxybenzoate; and
the photoinitiator comprises one or more of camphorquinone, bis(2,4,6-trimethylbenzoyl)phenylphosphine oxide, and ethyl 2,4,6-trimethylbenzoylphenylphosphinate.

5. The antibacterial resin mixture according to claim 1, wherein the polar organic solvent is an ester solvent;
optionally, the ester solvent comprises an acetate ester.

6. The antibacterial resin mixture according to claim 1, wherein based on 100% by mass of the second antibacterial agent, the second antibacterial agent further comprises 0.1% to 0.5% of triclosan.

7. The antibacterial resin mixture according to claim 1, wherein a preparation method for the coupling-modified nano-metal oxide comprises following steps: mixing a nano-metal oxide with a coupling agent and a solvent, and performing coupling modification, to obtain the coupling-modified nano-metal oxide;
optionally, the coupling agent comprises a silane coupling agent;
optionally, the nano-metal oxide comprises one or more of nano-zinc oxide, nano-silver oxide, nano-yttrium oxide, nano-lanthanum oxide, nano-magnesium oxide, nano-copper oxide, nano-alumina, and nano-iron oxide; and
optionally, a particle size of the nano-metal oxide ranges from 1 nm to 100 nm.

8. A preparation method for the antibacterial resin mixture according to any one of claims 1 to 7, **characterized by** comprising following steps: mixing the acrylate monomer, the initiator, and the first antibacterial agent or the second antibacterial agent to obtain the antibacterial resin mixture.

9. Use of the antibacterial resin mixture according to any one of claims 1 to 7 or the antibacterial resin mixture prepared by the preparation method according to claim 8 in preparation of a dental material.

10. The use according to claim 9, wherein the dental material comprises an antibacterial resin, an elastic ceramic material, or a resin-infiltrated ceramic composite material.

11. A resin-infiltrated ceramic composite material, **characterized by** comprising a porous ceramic framework and an antibacterial resin infiltrated into the porous ceramic framework, wherein the antibacterial resin is formed by curing the antibacterial resin mixture according to any one of claims 1 to 7 or the antibacterial resin mixture prepared by the preparation method according to claim 8.

12. The resin-infiltrated ceramic composite material according to claim 11, wherein a material of the porous ceramic framework comprises any one or a combination of at least two of aluminosilicate, feldspar, silica, zirconia, tungsten oxide, nepheline, and magnesium silicate.

13. The resin-infiltrated ceramic composite material according to claim 11, wherein the porous ceramic framework comprises a coupling-modified porous ceramic framework or a conventional porous ceramic framework.

14. A preparation method for the resin-infiltrated ceramic composite material according to any one of claims 11 to 13, **characterized by** comprising following steps: immersing the porous ceramic framework in the antibacterial resin mixture for vacuum infiltration, to infiltrate the antibacterial resin mixture into the porous ceramic framework, and performing curing, to obtain the resin-infiltrated ceramic material, wherein
the antibacterial resin mixture is the antibacterial resin mixture according to any one of claims 1 to 7 or the antibacterial resin mixture prepared by the preparation method according to claim 8.

15. The preparation method according to claim 14, wherein the immersing the coupling-modified porous ceramic framework in the antibacterial resin mixture for the vacuum infiltration comprises: immersing 1/3 of a height of the coupling-modified porous ceramic framework in the antibacterial resin mixture for first vacuum infiltration, supplementing the antibacterial resin mixture to 2/3 of the height of the porous ceramic framework for second vacuum infiltration, and continuously supplementing the antibacterial resin mixture to completely immerse the porous ceramic framework for third vacuum infiltration,
wherein the antibacterial resin mixture is prepared from the component (1).

16. The preparation method according to claim 15, wherein the first vacuum infiltration is carried out at pressure of 100 Pa to 1000 Pa for 24 h to 96 h; the second vacuum infiltration is carried out at pressure of 100 Pa to 1000 Pa for 24 h to 96 h; and the third vacuum infiltration is carried out at pressure of 0.1 Pa to 100 Pa for 24 h to 96 h.

17. An antibacterial resin, **characterized by** being obtained by curing an antibacterial resin mixture, wherein the antibacterial resin mixture is the antibacterial resin mixture according to any one of claims 1 to 7 or the antibacterial resin mixture prepared by the preparation method according to claim 8,
wherein the antibacterial resin mixture is prepared from the component (2).

18. An elastic ceramic material, **characterized by** comprising a matrix and a filler dispersed in the matrix, wherein the matrix is the antibacterial resin according to claim 17; and the filler comprises glass powder;
optionally, a mass content of the filler in the elastic ceramic material ranges from 60% to 80%; and
optionally, a particle size of the filler ranges from 1 µm to 30 µm.

19. A preparation method for the elastic ceramic material according to claim 18, **characterized by** comprising following steps: mixing the antibacterial resin mixture with the filler to obtain a resin body containing the filler; and
curing the resin body containing the filler to obtain the elastic ceramic material, wherein
the antibacterial resin mixture is the antibacterial resin mixture according to any one of claims 1 to 7 or the antibacterial resin mixture prepared by the preparation method according to claim 8.

20. A dental prosthesis, **characterized by** being made from the resin-infiltrated ceramic composite material according to claim 11, the antibacterial resin according to claim 17, or the elastic ceramic material according to claim 18, wherein the dental prosthesis comprises one or more of inlays, onlays, veneers, single crowns, and three-unit bridges,
wherein a conventional porous ceramic framework is used in the resin-infiltrated ceramic composite material according to claim 11.
